# EUROPEAN PATENT SPECIFICATION

(11) **EP 2 644 221 B1**
(45) Date of publication and mention of the grant of the patent: **31.12.2014**
(21) Application number: 13001548.0
(22) Date of filing: 26.03.2013
(51) Int. Cl.: A61M 16/04

(54) **Tracheostomy tube**
Tracheostomiekanüle
Tube de trachéostomie

(30) Priority: 29.03.2012 DE 102012006396
(43) Date of publication of application: 02.10.2013
(73) Proprietor: Willy Rüsch GmbH, 71394 Kernen-Rommelshausen (DE)
(72) Inventor: McBurney, Denzell, Castledaly, Moate Co Westmeath (IE); Nadason, Selvam, Penang (MY)
(74) Representative: Grünecker, Kinkeldey, Stockmair & Schwanhäusser

(56) References cited:
- EP-A2- 2 229 972
- WO-A1-2010/089523
- FR-A- 1 594 260
- GB-A- 2 056 285
- US-A- 2 198 241

## Description

The present invention relates to a tracheostomy tube with a tracheostomy cannula being provided at least in areas with a helical reinforcement and with at least one phonation opening disposed in the tracheostomy cannula.

Reinforced tracheostomy tubes have been produced for quite some time. The tracheostomy cannula is commonly made of a PVC material being correspondingly soft. The reinforcement acts as a support structure for the tracheostomy cannula and protects the tracheostomy tube from buckling and bending.

The tracheostomy tubes have drawbacks in that tracheostomy patients usually cannot speak because, due to the tracheostomy, the respiratory air no longer passes through the glottis but via the tracheostomy cannula past it. Meanwhile, there are ways of also enabling speech for people who have a permanently affixed tracheostomy tube and who still possess speech functionality. This requires that a significant portion of the exhaled air be diverted past the tracheostomy cannula through the glottis. This is done with a one-way valve that is mounted externally to the tracheostomy tube. This valve allows the patient to inhale through the tube, but closes when the patient wishes to exhale. The expiratory air thus flows past the tracheostomy cannula over the glottis. The spacing between the outer surface of the tracheostomy tube and the inner lumen of the trachea, however, is usually very small, so that the patient must exert a relatively high amount of pressure to squeeze the exhaled air through the glottis for phonation. Breathing effort is therefore increased, which is undesirable.

Tracheostomy tubes have therefore been developed as an alternative, which have a phonation opening on the greater curvature of the tracheostomy cannulae. The phonation opening may take the shape of a single large opening interrupting the wall of the tube. Furthermore, several small phonation openings may be provided, which also interrupt the wall of the tracheostomy tube. Until now, these openings have been known only in relatively rigid tracheostomy tubes. These rigid tracheostomy tubes need no reinforcement, because they do not collapse due to their high inherent rigidity. Furthermore, helically reinforced highly flexible tracheostomy tubes comprising phonation openings are known, in which the reinforcement is interrupted and the phonation opening is arranged in this interruption between the two ends of the helical reinforcement. The drawback here is that there is no stabilization of the tracheostomy tube provided in the area of the phonation opening so that buckling or bending cannot be prevented.

Document WO2010/089523 shows a tracheostomy cannula with a helical reinforcement. Document US2198241 shows a tracheo-stomy cannula with a fenestration.

It is therefore an objective of the present invention to provide a tracheostomy tube which prevents the drawbacks of the above-mentioned prior art, and in particular enables stabilization of the tracheostomy cannula in all essential areas.

For this purpose, the invention provides that the pitch of the helical reinforcement in the area of the at least one phonation opening is increased such that the at least one phonation opening is fitted in-between two turns of the helical reinforcement.

In this manner, the tracheostomy cannula is therefore also supported by reinforcement in the area in which the phonation opening is disposed. High stability of the tracheostomy cannula and thus of the tracheostomy tube is reached in this manner, any buckling or bending of the tracheostomy cannula is prevented.

It can be provided in an advantageous embodiment, that the helical reinforcement is fully embedded into the tracheostomy cannula. The at least one phonation opening is therefore not crossed by the reinforcement, so that the free cross-section of the phonation opening is not restricted. Since the reinforcement is not exposed, the surfaces of the tracheostomy cannula are smooth, so that discharging body fluids or particles at the reinforcement is prevented. Cleaning the tracheostomy tube is facilitated.

A further advantageous embodiment can provide that the helical reinforcement is formed throughout. The reinforcement is therefore not interrupted in the area of the phonation opening. High stability is thus warranted.

It may also be provided that the tracheostomy cannula comprises a curve, so that the turns of the helical reinforcement are compressed at the inner radius of the curve and are bent open at the outer radius of the curve, and that the at least one phonation opening is arranged at the outer radius of the curve. By bending up the reinforcement at the outer radius of the curve, room for the phonation opening is advantageously created so that unobstructed outflow of the exhaled air in the direction of the glottis is enabled.

Advantageously, multiple phonation openings can further be provided which are disposed between the turns of the helical reinforcement. If several phonation openings are provided, then the distance between the individual turns of the helical reinforcement must be increased only to a small degree. Higher stability is achieved. By having several small openings being formed in the tracheostomy cannula, the surface is smoother, insertion of the tracheostomy tube into the trachea is facilitated.

Yet another embodiment may provide that the pitch of the helical reinforcement in the area in which the at least one phonation opening is disposed, is about six times the minimum pitch of the reinforcement. It has been shown, that this achieves good stability of the tracheostomy tube at simultaneously an advantageous size of the phonation openings.

Yet another embodiment provides that the total free area of all phonation openings be in a range from about 30 to 125 mm². This ensures that sufficient air flows through the phonation opening in the direction of the glottis, so that speaking is possible without too much increasing breathing work.

It has been shown that good stability of the tracheostomy tube can be achieved, if the length of the area with increased pitch of the helical reinforcement, in which the at least one phonation opening is disposed, is approximately 14 to 18% of the total length of the tracheostomy cannula.

It can also be provided that both ends of the tracheostomy cannula are without reinforcement. The ends of the tracheostomy tube or the tracheostomy cannula, respectively, are thereby softer, thus facilitating inserting the tracheostomy tube into the trachea and fitting valves and caps.

It can advantageously also be provided that an inner cannula comprising at least one phonation opening being aligned with the at least one phonation opening of the tracheostomy cannula is disposed in the tracheostomy cannula. The inner cannula facilitates cleaning of the tracheostomy tube and thus extends the time that the tracheostomy tube is in the patient.

It can be provided according to still another embodiment, that a cuff is attached to the end of the tracheostomy cannula to be disposed in the trachea. In this manner, the end of the tracheostomy cannula is sealed off from the trachea, any reverse flow of body fluids into the trachea is prevented and the desired speech function via the phonation openings is ensured.

The invention is further illustrated in more detail using the drawings. They show in:
Fig. 1 tracheostomy tube with reinforced tracheostomy cannula provided with phonation openings,
Fig. 2 tracheostomy cannula with phonation openings in a stretched state,
Fig. 3 magnification of the tracheostomy cannula of Fig. 2 in the area of the phonation openings,
Fig. 4 tracheostomy cannula in a bent state and
Fig. 5 magnification of the curve of the tracheostomy cannula of Fig. 4.

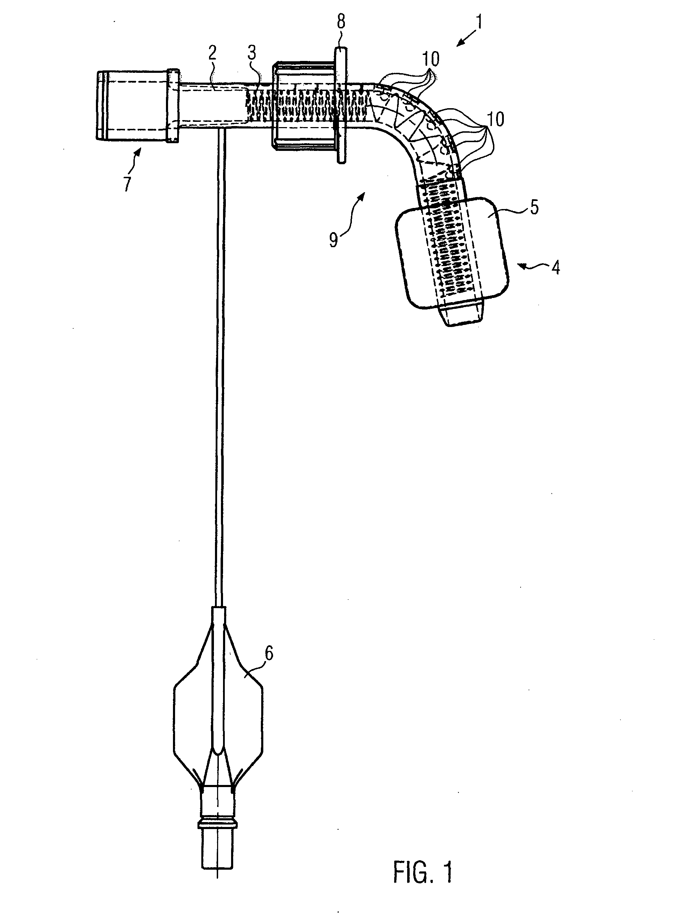

Fig.1 shows a tracheostomy tube 1 according to the invention. The tracheostomy tube 1 comprises a tracheostomy cannula 2, in which a reinforcement 3 is embedded. The reinforcement 3 is formed in a helical manner. Metal wire can for instance be used as material for the reinforcement. There is no reinforcement provided at the tracheostomy cannula's 2 end 4 to be disposed in the trachea nor at the opposite end 7.

The tracheostomy cannula 2 comprises a curve 9, which is designed such that the tracheostomy tube's 1 end 4 to be disposed in the trachea and the opposite end 7 of the tracheostomy tube 1 lock with each other in an angle of approximately 80°-100°; preferably a right angle. In the tracheostomy cannula 2, phonation openings 10 are formed at the outer radius of the curve 9. The phonation openings 10 penetrate the wall of the tracheostomy cannula 2. The reinforcement 3 of the tracheostomy cannula 2 is here formed such that, in the area where the phonation openings 10 are disposed, the spacing between the turns of the reinforcement 3 at least corresponds to the diameter of a phonation opening 10.

A cuff 5 is disposed at the tracheostomy tube's 1 end 4 to be disposed in the trachea. The cuff 5 is connected by means of a line (not shown) with a balloon 6. If the tracheostomy tube is in use and disposed in the trachea of a patient, then the cuff 5 can be inflated by means of the external balloon 6 and a sealing the end 4 of the tracheostomy tube 1 in the trachea can be achieved. At the other end 7 of the tracheostomy tube 1, i.e. at the end which comes to lie at the neck of the patient outside of the trachea, a holding plate 8 is attached. The tracheostomy tube can be affixed to the neck of a patient by means of this holding plate 8. Straps are commonly used for this. In addition, various valves or caps, respectively, can be mounted onto this second end of the tracheostomy tube 1, for example, a speech valve, a cough lid, a cap, or a connector. An inner cannula can be disposed in the interior of the tracheostomy cannula 2. In this manner, cleanability of the tracheostomy tube 1 is facilitated since only the inner cannula needs to be removed and cleaned or replaced, respectively. Usage time of a tracheostomy tube can thus be extended.

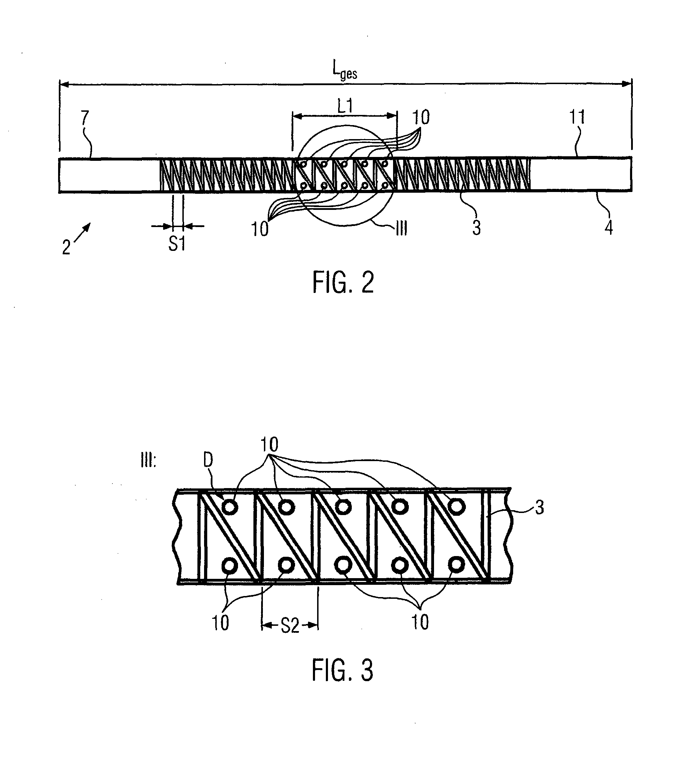

Fig. 2 shows a tracheostomy cannula 2 in a stretched state, i.e. before it is brought into the bent state desired for the tracheostomy tube 1. The tracheostomy cannula 2 comprises a plastic tube 11, in which reinforcement 3 is embedded. The reinforcement is helically shaped and preferably made of metal wire. Usually these tracheostomy cannulae are produced by an immersion method. In this, a form is immersed into liquid PVC paste and then dried (gelification). After that, the helical reinforcement is drawn across the resulting dried pre-dip and in further immersion steps the helical metal reinforcement is overdipped and the wall thickness of the cannula is increased to the final dimension. The reinforcement 3 is thereby fully embedded in the tracheostomy cannula 2.

The reinforcement 3 is designed to be formed continuously and therefore has no interruptions. This ensures that an adequate support effect is achieved in the sensitive central area of the tracheostomy cannula 2 and the tracheostomy cannula does not buckle or bend. By buckling or bending, the diameter of the tracheostomy cannula 2 is reduced or completely closed, which impedes or prevents breathing, which must be prevented. The phonation openings 10 are disposed in a central area of the tracheostomy cannula 2, preferably in the area where the tracheostomy cannula 2 curves. For this, the pitch S2 of the helical reinforcement 3 is increased in this area. The pitch S2 is at least large enough that the distance between two adjacent turns of the reinforcement 3 corresponds at least to the diameter of a phonation opening. Thus, at least one phonation opening fits in-between two turns of the helical reinforcement 3, without the diameter of the helical phonation opening being crossed by the helical reinforcement. In the other areas of the helical reinforcement, the pitch S1 is smaller than in the area of the phonation openings 10. As shown in Fig. 2, the pitch in these other areas of the helical reinforcement 3 can be unchanged. It would also be conceivable, however, that variations in the pitch of the helical reinforcement are provided in these areas.

In the example illustrated, the pitch S2 of the helical reinforcement 3 in the area of the phonation opening 10 is about six times the pitch S1 of the helical reinforcement 3 in the remaining areas. The length L1 of the area in which the pitch S2 of the helical reinforcement 3 is increased equals about 14 to 18% of the total length L_{ges} of the tracheostomy cannula 2.

Fig. 3 shows a magnification of the area of the tracheostomy cannula 2 in which the phonation openings 10 are disposed, from Fig. 2. The helical reinforcement 3 is in this area also designed to be formed continuously. This achieves increased stability of the tracheostomy cannula 2 also in this area. The pitch S2 of the helical reinforcement 3 is designed such that the spacing between the individual turns of the helical reinforcement 3 is increased, so that it corresponds at least to the diameter D of a phonation opening 10. The diameter D of a phonation opening 10 is in a range from about 2 to 4 mm depending on the size of the tracheostomy tube 1. In the case illustrated, ten phonation openings are provided. This ensures that the diameter of the individual phonation openings does not become too large and that sufficient free total area of the phonation openings is still available. The total free area of all phonation openings 10 presently lies in a range from about 30 to 125 mm² depending on the size of the tracheostomy tube 1.

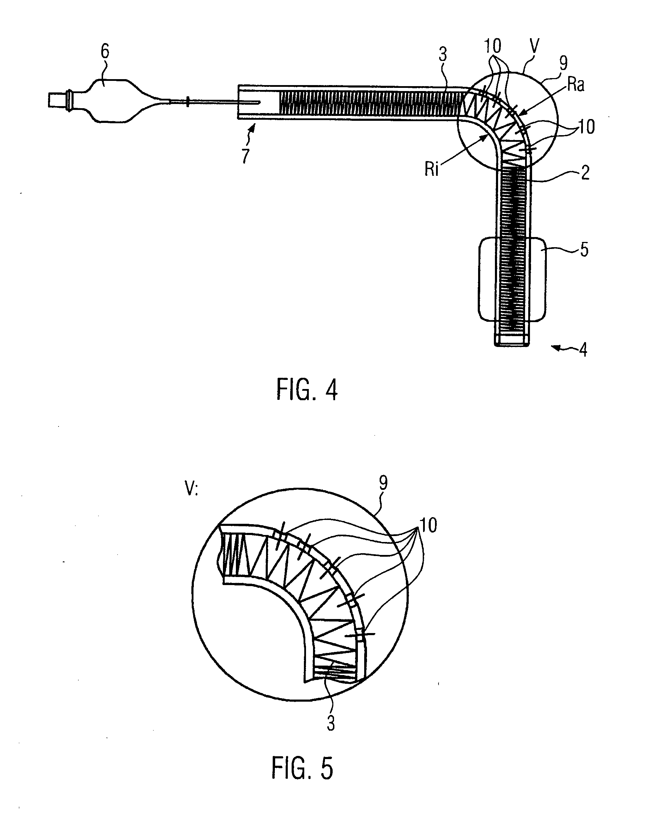

Fig. 4 shows the tracheostomy cannula 2 in a curved state. The holding plate and possibly mounted caps or valves are not shown. The tracheostomy cannula 2 comprises a plastic tube in which helical reinforcement 3 is completely embedded. The tracheostomy cannula 2 thus has smooth surfaces, so that accumulation of particles and fluids is reduced. At the tracheostomy tube's 1 end 7, which during use is disposed at the neck of the patient, the balloon 6 is shown which is connected via a line, not shown, with the cuff 5 and enables inflating the cuff for sealing. Since the reinforcement 3 of the tracheostomy cannula 2 is formed helically, the reinforcement 3 in the area of the curve 9 of the tracheostomy cannula 2 is compressed in the inner radius and spread out at the outer radius. The phonation openings 10 are disposed in the area of the outer radius Ra. By spreading open the reinforcement 3, there is sufficient space for the phonation openings 10.

There is no reinforcement provided at the tracheostomy cannula's 2 end 4 to be disposed in the trachea nor at the opposite end 7. Therefore, the tracheostomy cannula 2 is softer in this area. This enables easier attachment of the valves or caps and prevents injuries during insertion of the tracheostomy tube 1 into the trachea of a patient.

Fig. 5 illustrates in magnification the area of the curve 9 of the tracheostomy cannula 2 of Fig. 4. In this area, the helical reinforcement 3 has the increased pitch S2, so that the phonation openings 10 can be disposed between the individual turns of the helical reinforcement 3. The phonation openings 10 are located at the outer radius Ra of the curve 9. By means of the curve 9, the turns of the helical reinforcement 3 are additionally spread open, so that sufficient space for the phonation openings 10 is created. Fig. 5 again shows that the pitch of the helical reinforcement 3 is smaller in the areas before and behind the phonation openings than in the area of the phonation openings 10.

## Claims

1. Tracheostomy tube (1) with a tracheostomy cannula (2) being provided at least in areas with helical reinforcement (3) and with at least one phonation opening (10) disposed in said tracheostomy cannula (2), **characterized in that** the pitch (S2) of said helical reinforcement (3) in the area of the at least one phonation opening (10) is increased such that the at least one phonation opening (10) is fitted between two turns of said helical reinforcement (3).

2. Tracheostomy tube according to claim 1, **characterized in that** said helical reinforcement (3) is fully embedded in said tracheostomy cannula (2).

3. Tracheostomy tube according to claim 1 or 2, **characterized in that** said helical reinforcement (3) is formed continuously.

4. Tracheostomy tube according to one of the claims 1 to 3, **characterized in that** said tracheostomy cannula (2) comprises a curve (9) so that the turns of said helical reinforcement (3) are compressed at the inner radius (Ri) of said curve (9) and are bent open at the outer radius (Ra) of said curve, and that said at least one phonation opening (10) is disposed at said outer radius (Ra) of said curve (9).

5. Tracheostomy tube according to one of the claims 1 to 4, **characterized in that** multiple phonation openings (10) are provided, which are disposed between said turns of said helical reinforcement (3).

6. Tracheostomy tube according to one of the claims 1 to 5, **characterized in that** the pitch (S2) of said helical reinforcement (3) in the area in which the at least one phonation opening (10) is disposed is about six times the minimum pitch (S1).

7. Tracheostomy tube according to one of the claims 1 to 6, **characterized in that** the total free area of all phonation openings (10) is in a range from about 30 to 125 mm².

8. Tracheostomy tube according to one of the claims 1 to 7, **characterized in that** the length (L1) of said area with increased pitch (S2) of said helical reinforcement (3) in which said at least one phonation opening (10) is disposed, is approximately 14 to 18% of the total length (L_{ges}) of said tracheostomy cannula (2).

9. Tracheostomy tube according to one of the claims 1 to 8, **characterized in that** both ends (4, 7) of the tracheostomy cannula (2) are without reinforcement.

10. Tracheostomy tube according to one of the claims 1 to 9, **characterized in that** an inner cannula comprising at least one phonation opening, being aligned with said at least one phonation opening (10) of said tracheostomy cannula (2), is disposed in said tracheostomy cannula (2).

11. Tracheostomy tube according to one of the claims 1 to 10, **characterized in that** a cuff (5) is attached to said tracheostomy cannula's (2) end (4) to be disposed in the trachea.

## Patentansprüche

1. Tracheostomietubus (1), mit einer zumindest bereichsweise mit einer wendelförmig Armierung (3) versehenen Tracheostomiekanüle (2) und mit mindestens einer, in der Tracheostomiekanüle (2) angeordneten Phonationsöffnung (10), **dadurch gekennzeichnet, dass** die Steigung (S2) der wendelförmigen Armierung (3) im Bereich der mindestens einen Phonationsöffnung (10) vergrößert ist, derart, dass die mindestens eine Phonationsöffnung (10) zwischen zwei Windungen der wendelförmigen Armierung (3) eingepasst ist.

2. Tracheostomietubus nach Anspruch 1, **dadurch gekennzeichnet, dass** die wendelförmige Armierung (3) vollständig in die Tracheostomiekanüle (2) eingebettet ist.

3. Tracheostomietubus nach Anspruch 1 oder 2, **dadurch gekennzeichnet, dass** die wendelförmige Armierung (3) durchgehend ausgebildet ist.

4. Tracheostomietubus nach einem der Ansprüche 1 bis 3, **dadurch gekennzeichnet, dass** die Tracheostomiekanüle (2) eine Biegung (9) aufweist, so dass die Windungen der wendelförmigen Armierung (3) am Innenradius (Ri) der Biegung (9) zusammengedrückt und am Außenradius (Ra) der Biegung aufgebogen werden, und dass die mindestens eine Phonationsöffnung (10) am Außenradius (Ra) der Biegung (9) angeordnet ist.

5. Tracheostomietubus nach einem der Ansprüche 1 bis 4, **dadurch gekennzeichnet, dass** mehrere Phonationsöffnungen (10) vorgesehen sind, die zwischen den Windungen der wendelförmigen Armierung (3) angeordnet sind.

6. Tracheostomietubus nach einem der Ansprüche 1 bis 5, **dadurch gekennzeichnet, dass** die Steigung (S2) der wendelförmigen Armierung (3) in dem Bereich, in dem die mindestens eine Phonationsöffnung (10) angeordnet ist, etwa das sechsfache der Minimalsteigung (S1) beträgt.

7. Tracheostomietubus nach einem der Ansprüche 1 bis 6, **dadurch gekennzeichnet, dass** die freie Gesamtfläche aller Phonationsöffnungen (10) in einem Bereich von 30 bis 125 mm² liegt.

8. Tracheostomietubus nach einem der Ansprüche 1 bis 7, **dadurch gekennzeichnet, dass** die Länge (L1) des Bereichs mit vergrößerter Steigung (S2) der wendelförmigen Armierung (3), in dem die mindestens eine Phonationsöffnung (10) angeordnet ist, etwa 14 bis 18 % der Gesamtlänge (L_{ges}) der Tracheostomiekanüle (2) beträgt.

9. Tracheostomietubus nach einem der Ansprüche 1 bis 8, **dadurch gekennzeichnet, dass** beide Enden (4, 7) der Tracheostomiekanüle (2) armierungsfrei sind.

10. Tracheostomietubus nach einem der Ansprüche 1 bis 9, **dadurch gekennzeichnet, dass** in der Tracheostomiekanüle (2) eine Innenkanüle angeordnet ist, welche mindestens eine Phonationsöffnung aufweist, die mit der mindestens einen Phonationsöffnung (10) der Tracheostomiekanüle (2) fluchtet.

11. Tracheostomietubus nach einem der Ansprüche 1 bis 10, **dadurch gekennzeichnet, dass** an dem in der Luftröhre anzuordnenden Ende (4) der Tracheostomiekanüle (2) ein Cuff (5) angebracht ist.

## Revendications

1. Tube de trachéotomie (1) comportant une canule de trachéotomie (2) pourvue d'un renfort hélicoïdal (3) au moins dans certaines zones et d'au moins un orifice de phonation (10) réalisé dans ladite canule de trachéotomie (2), **caractérisé en ce que** le pas (S2) dudit renfort hélicoïdal (3) dans la zone dudit au moins un orifice de phonation (10) est augmenté de telle sorte que ledit au moins un orifice de phonation (10) est réalisé entre deux tours dudit renfort hélicoïdal (3).

2. Tube de trachéotomie selon la revendication 1, **caractérisé en ce que** ledit renfort hélicoïdal (3) est entièrement intégré dans ladite canule de trachéotomie (2).

3. Tube de trachéotomie selon la revendication 1 ou 2, **caractérisé en ce que** ledit renfort hélicoïdal (3) est formé de manière continue.

4. Tube de trachéotomie selon l'une des revendications 1 à 3, **caractérisé en ce que** ladite canule de trachéotomie (2) comprend une courbe (9) de telle sorte que les tours dudit renfort hélicoïdal (3) sont compressés sur le rayon interne (Ri) de ladite courbe (9) et sont pliés pour s'ouvrir sur le rayon externe (Ra) de ladite courbe, et **en ce que** ledit au moins un orifice de phonation (10) est agencé sur ledit rayon externe (Ra) de ladite courbe (9).

5. Tube de trachéotomie selon l'une des revendications 1 à 4, **caractérisé en ce que** de multiples ouvertures de phonation (10) sont pourvues et agencées entre lesdits tours dudit renfort hélicoïdal (3).

6. Tube de trachéotomie selon l'une des revendications 1 à 5, **caractérisé en ce que** le pas (S2) dudit renfort hélicoïdal (3) dans la zone dans laquelle ledit au moins un orifice de phonation (10) est agencé est environ six fois le pas minimum (S1).

7. Tube de trachéotomie selon l'une des revendications 1 à 6, **caractérisé en ce que** la surface libre totale de tous les orifices de phonation (10) est comprise entre environ 30 mm² et 125 mm².

8. Tube de trachéotomie selon l'une des revendications 1 à 7, **caractérisé en ce que** la longueur (L1) de ladite zone à pas augmenté (S2) dudit renfort hélicoïdal (3) dans laquelle ledit au moins un orifice de phonation (10) est agencé correspond à approximativement 14 % à 18 % de la longueur totale (L_{ges}) de ladite canule de trachéotomie (2).

9. Tube de trachéotomie selon l'une des revendications 1 à 8, **caractérisé en ce que** les deux extrémités (4, 7) de la canule de trachéotomie (2) ne comportent pas de renforcement.

10. Tube de trachéotomie selon l'une des revendications 1 à 9, **caractérisé en ce qu'**une canule interne comprenant au moins un orifice de phonation, qui est aligné avec ledit au moins un orifice de phonation (10) de ladite canule de trachéotomie (2), est agencée dans ladite canule de trachéotomie (2).

11. Tube de trachéotomie selon l'une des revendications 1 à 10, **caractérisé en ce qu'**un manchon (5) est attaché à l'extrémité (4) de ladite canule de trachéotomie (2) à agencer dans la trachée.
